# EUROPÄISCHE PATENTSCHRIFT

(11) **EP 2 751 064 B1**
(45) Veröffentlichungstag und Bekanntmachung des Hinweises auf die Patenterteilung: **10.08.2016**
(21) Anmeldenummer: 12762554.9
(22) Anmeldetag: 29.08.2012
(51) Int. Cl.: C07C 201/08, C07C 201/16, C07C 209/36, C07C 205/06, C07C 211/46

(54) **VERFAHREN ZUR KONTINUIERLICHEN HERSTELLUNG VON NITROBENZOL**
METHOD FOR CONTINUOUS PRODUCTION OF NITROBENZENE
PROCÉDÉ DE PRODUCTION EN CONTINU DE NITROBENZÈNE

(30) Priorität: 31.08.2011 DE 102011081898
(43) Veröffentlichungstag der Anmeldung: 09.07.2014
(73) Patentinhaber: Covestro Deutschland AG, 51373 Leverkusen (DE)
(72) Erfinder: KNAUF, Thomas, 41542 Dormagen (DE); MERKEL, Michael, 40223 Düsseldorf (DE)
(74) Vertreter: Levpat
(86) Internationale Anmeldenummer: PCT/EP2012/066751
(87) Internationale Veröffentlichungsnummer: WO 2013/030223

(56) Entgegenhaltungen:
- EP-A1- 0 011 090
- EP-A1- 1 816 117
- DE-T2- 60 113 579

## Beschreibung

Die Erfindung betrifft ein Verfahren zur Herstellung von Nitrobenzol, bei dem man zunächst durch Nitrierung von Benzol Roh-Nitrobenzol herstellt, welches dann nacheinander in mindestens einer sog. sauren Wäsche, in mindestens einer alkalischen Wäsche und in mindestens einer neutralen Wäsche gewaschen wird, wobei man zwischen der letzten alkalischen Wäsche und der ersten neutralen Wäsche mindestens eine zusätzliche Wäsche mit einer wässrigen Lösung eines Kaliumsalzes zwischenschaltet.

Nitrobenzol ist ein wichtiges Zwischenprodukt der chemischen Industrie, das insbesondere zur Herstellung von Anilin und damit auch zur Herstellung der Di- und Polyisocyanate der Diphenylmethanreihe und den darauf basierenden Polyurethanen benötigt wird.

Die Nitrierung von Benzol mit Salpetersäure zu einem Roh-Nitrobenzol war bereits Gegenstand zahlreicher Veröffentlichungen und Patentanmeldungen. Die heute gängigen Verfahren entsprechen im Wesentlichen dem Konzept der adiabaten Nitrierung von Benzol durch ein Gemisch von Schwefel- und Salpetersäure (sog. *Mischsäure*). Ein solches Verfahren wurde erstmals in US 2,256,999 beansprucht und ist in heutigen Ausführungsformen beispielsweise in EP 0 436 443 B1, EP 0 771 783 B1 und US 6 562 247 B2 beschrieben. Die Verfahren mit adiabater Reaktionsführung zeichnen sich insbesondere dadurch aus, dass keine technischen Maßnahmen ergriffen werden, um der Reaktionsmischung Wärme zu- oder abzuführen.

Auch sind isotherme Verfahren zur Nitrierung von Benzol mit Mischsäure beschrieben, wie beispielsweise in EP 0 156 199 B1 beschrieben.

Auch sind Verfahren zur Nitrierung von Benzol bekannt, die ohne den Einsatz von Schwefelsäure auskommen. Diese sind beispielsweise beschrieben in US 2 739 174 oder US3 780 116.

Grundsätzlich sind auch Gasphasenverfahren zur Nitrierung von Benzol mit Salpetersäure oder Stickstoffoxiden möglich, jedoch sind die dadurch erzielbaren Ausbeuten noch gering (EP 0 078 247B1, EP 0 552 130 B1).

All diesen Verfahren ist gemeinsam, dass als Reaktionsprodukt zunächst ein Roh-Nitrobenzol entsteht, das als Verunreinigungen Salpetersäure und - sofern mit Mischsäure nitriert wurde - Schwefelsäure enthält, sowie als organische Verunreinigungen Dinitrobenzol sowie nitrierte Oxidationsprodukte des Benzols, insbesondere nitrierte Phenole (Nitrophenole). Auch enthält es organische Verbindungen, die aus den Verbindungen entstanden, die als Verunreinigungen im eingesetzten Benzol enthalten waren (WO 2008/148608 A1). Darüber hinaus enthält das RohNitrobenzol auch Metallsalze, die in den Säureresten oder im Roh-Nitrobenzol gelöst vorliegen können (DE 10 2007 059 513 A1).

Zahlreiche Untersuchungen zielten in der Vergangenheit darauf ab, die Qualität des Roh-Nitrobenzols zu verbessern und somit die Ausbeute bezüglich Benzol und Salpetersäure zu erhöhen. Dank dieser Entwicklungen sind die heutigen adiabaten Flüssigphasenverfahren so ausgereift, dass es allen gelingt, ein Roh-Nitrobenzol herzustellen, das einen geringen Gehalt an Nebenprodukten aufweist, also im Allgemeinen nur 100 ppm bis 300 ppm an Dinitrobenzol und 1500 ppm bis 2500 ppm an Nitrophenolen enthält, wobei Pikrinsäure einen Anteil von 10 % bis 50 % an den Nitrophenolen annehmen kann.

Das Roh-Nitrobenzol weist als Verunreinigungen noch Wasser, Benzol sowie Nitrophenole und Dinitrobenzol und - sofern mit Mischsäure nitriert wurde - Schwefelsäure auf. Diese Verunreinigungen sind unerwünscht, da sie in Nachfolgeprozessen, wo Nitrobenzol zum Einsatz kommt, wie zum Beispiel der Herstellung von Anilin, diese negativ beeinflussen können. Geeignete Aufarbeitungsverfahren, die Wasch- und Destillationsstufen beinhalten, sind, z. B. in US 6,288,289 B1, EP 1 593 654 A1, EP 1 816 117 B1 und WO2011/021057 A1beschrieben.

In EP 1 816 117 B1 wird die Aufarbeitung des Roh-Nitrobenzols in einer sauren Wäsche, einer alkalischen Wäsche mit wässriger Natronlauge, einer neutralen Wäsche und einer abschließenden destillativen Reinigung beschrieben. Natürlich können grundsätzlich auch andere Basen als Natronlauge, wie zum Beispiel wässrige Natriumcarbonatlösung oder wässrige Ammoniaklösung **(**WO 2011/082 977 A1) oder Kaliumhydroxid oder Ammoniak (DE 60 113 579 T2) verwendet werden.

Eine andere Ausführungsform der Aufarbeitung von Roh-Nitrobenzol ist in WO2011/021057 A1 beschrieben, wo im Detail auf die Salzproblematik im Waschprozess eingegangen wird. Das Roh-Nitrobenzol wird im ersten Schritt mit Wasser gewaschen, anschließend einer alkalischen Wäsche mit Natronlauge unterzogen und abschließend sauer gewaschen bevor es einer Dampfstrippung unterzogen wird, um Wasser und überschüssiges Benzol zu entfernen. Die bevorzugte Säure ist Salpetersäure, die bereits im Nitrier-Prozess vorkommt und flüchtig ist, so dass sie in der Dampfstrippung aus dem Produkt entfernt wird. Diese Ausführungsform hat den Nachteil, dass eine gewisse Menge Natronlauge und Natriumnitrophenolate aus der alkalischen Wäsche in die nächste Waschstufe - hier eine saure Wäsche - gespült werden. Bei der Umsetzung von Natriumnitrophenolaten mit Salpetersäure werden Nitrophenole freigesetzt, die wiederum als Verunreinigung in das Roh-Nitrobenzol gelangen. Ein Ziel der sauren Wäsche, diese Verbindungen vollständig aus dem Produkt zu entfernen, wird so nicht mehr erfüllt.

Das gereinigte Nitrobenzol (Rein-Nitrobenzol) wird überwiegend zur Herstellung von Anilin eingesetzt, die heute wiederum überwiegend durch katalytische Hydrierung des Nitrobenzols in der Gasphase mit Wasserstoff erfolgt. Zur Überführung in die Gasphase kann Nitrobenzol entweder verdampft (EP 0 696 574 B1, Absatz [0024]) oder in einen heißen Gasstrom hinein, bevorzugt in einen Wasserstoffstrom hinein verdüst (DE-OS-1 809 711, DE 10 2006 035 203 A1, Absatz [0053]) werden. Der Einsatz einer Verdampfung gilt als vorteilhaft, da sich dadurch deutlich geringere Ablagerungen im Reaktor und in den Zuleitungen bilden sollen (EP 0 696 574 B1, Absatz [0024]). Im Nitrobenzol befindliche Metallverbindungen, Salze und Hochsieder bleiben vielmehr im Verdampfer zurück und gelangen nicht in das Reaktionssystem. Der apparative Aufwand zur Verdampfung großer Mengen Nitrobenzol ist jedoch erheblich, so dass vielerorts eine Verdüsung zum Einsatz kommt. Hierbei wird das Nitrobenzol im Kreisgasstrom der Hydrieranlage verdüst, und so gelangen im Nitrobenzol befindliche Metallverbindungen, Salze und Hochsieder in den Reaktor. Unerwünschte Ablagerungen sind die Folge, so dass sich die Reinigungsintervalle des Reaktors verkürzen und Katalysator vorzeitig desaktiviert werden kann. In besonderem Maße schädlich sind solche Salze, die den Katalysator vergiften, da hier schon sehr kleine Mengen ausreichen, um eine Desaktivierung herbeizuführen.

Es bestand daher ein Bedarf an einem Verfahren zur Herstellung von Nitrobenzol, das Nitrobenzol in einer solchen Qualität bereitstellt, welche in einem Anilin-Gasphasenprozess auch bei Anwendung der Verdüsungstechnik zur Überführung des Nitrobenzols in die Gasphase nicht zu Beeinträchtigungen der Betriebsstabilität und / oder der Katalysatoraktivität führt. Weiterhin sollte der Nitrierprozess selbst möglichst störungsfrei (kein Anlagenausfall etwa durch Emulsionsbildung in der neutralen Wäsche) durchgeführt werden können.

Diesem Bedarf Rechnung tragend ist ein Gegenstand der vorliegenden Erfindung ein Verfahren zur Herstellung von Nitrobenzol durch
a) Nitrierung von Benzol mit Salpetersäure oder Gemischen aus Salpetersäure und Schwefelsäure und anschließende Phasentrennung in eine wässrige und eine organische, Nitrobenzol enthaltende Phase,
b) Waschen der in Schritt a) erhaltenen organischen, Nitrobenzol enthaltenden Phase in mindestens einer, bevorzugt einer bis zwei, besonders bevorzugt einer Wäsche(n) (sog. *"saure Wäsche(n)* ") und anschließende Phasentrennung in eine wässrige und eine organische, Nitrobenzol enthaltende Phase,
c) Waschen der in Schritt b) erhaltenen organischen, Nitrobenzol enthaltenden Phase in mindestens einer, bevorzugt einer bis zwei, besonders bevorzugt einer alkalischen Wäsche(n) mit einer wässrigen Lösung einer Base ausgewählt aus der Gruppe bestehend aus Natriumhydroxid, Natriumcarbonat und Natriumhydrogencarbonat,
   und anschließende Phasentrennung in eine wässrige und eine organische, Nitrobenzol enthaltende Phase,
d) Waschen der in Schritt c) erhaltenen organischen, Nitrobenzol enthaltenden Phase in mindestens einer, bevorzugt einer bis zwei, besonders bevorzugt einer Wäsche(n) mit einer wässrigen Lösung eines Kaliumsalzes und anschließende Phasentrennung in eine wässrige und eine organische, Nitrobenzol enthaltende Phase,
e) Waschen der in Schritt d) erhaltenen organischen, Nitrobenzol enthaltenden Phase in mindestens einer, bevorzugt zwei bis vier, besonders bevorzugt zwei bis drei, ganz besonders bevorzugt zwei neutralen Wäsche(n) mit Wasser und anschließende Phasentrennung in eine wässrige und eine organische, Nitrobenzol enthaltende Phase,
f) Aufarbeitung der in Schritt e) erhaltenen, organischen, Nitrobenzol enthaltenden Phase, wobei gereinigtes Nitrobenzol erhalten wird.

Ein weiterer Gegenstand der vorliegenden Erfindung ist ein Verfahren zur Herstellung von Anilin, bei dem man Nitrobenzol erfindungsgemäß herstellt und in Gegenwart eines Katalysators zu Anilin hydriert.

Es wurde gefunden, dass der Einsatz von Nitrobenzol, das gelöste Natriumsalze enthält, zu signifikanten Beeinträchtigungen eines Gasphasen-Anilinverfahrens insbesondere dann führt, wenn das Nitrobenzol nicht verdampft, sondern in den Kreisgasstrom verdüst wird. Die Ursache hierfür ist wahrscheinlich auf eine Schädigung des Katalysators durch in den Reaktor eingetragene Natriumsalze zurückzuführen. Überraschend wurde gefunden, dass dieser negative Effekt von Natriumsalzen bei Kaliumsalzen nicht oder zumindest nicht im gleichen Umfang auftritt.

Prinzipiell ließe sich der geschilderte nachteilige Effekt also vermeiden, wenn in der alkalischen Wäsche des Roh-Nitrobenzols an Stelle von Natronlauge ausschließlich Kalilauge eingesetzt würde. Da Kalilauge jedoch erheblich teurer ist als Natronlauge, ist diese Lösung wirtschaftlich wenig attraktiv. Eine wesentlich wirtschaftlichere Lösung des Problems lässt sich erreichen, wenn die alkalische Wäsche wie im Stand der Technik üblich mit Natronlauge durchgeführt und dann im Nitrobenzol gelöste Natriumionen durch Kaliumionen verdrängt werden. Typische Salze, die auf diese Weise im Nitrobenzol abgereichert werden können, sind Natrium-Nitrit, -Nitrat, -Sulfat (falls mit Mischsäure nitriert wird) und -Oxalat (entstanden durch Oxidationsreaktionen organischer Komponenten). Neben Natriumsalzen können auch andere Salze durch Kaliumsalze verdrängt werden, etwa die Calcium-Salze der vorgenannten Anionen. Calcium kann z. B. über verunreinigte Salpetersäure, wie in EP 2 070 907 A1 beschrieben, in das Nitrobenzol gelangen. Diese Verdrängung unerwünschter Salze wird durch den erfindungsgemäßen Schritt d) erreicht. Der Vorteil dieser Vorgehensweise besteht darin, dass in der Schritt d) vorausgehenden Phasentrennung bereits ein beträchtlicher Teil der Natriumionen mit der wässrigen Phase ausgetragen wird, sodass nur noch ein kleiner Teil der insgesamt über die Natronlauge eingebrachten Natriumionen durch einen Überschuss an Kaliumionen verdrängt werden muss. Hierdurch wird die insgesamt einzusetzende Menge an Kaliumsalz gegenüber einer Fahrweise, bei der die alkalische(n) Wäsche(n) komplett mit Kalilauge betrieben wird/werden, erheblich reduziert.

Nachfolgend werden Ausführungsformen der Erfindung näher beschrieben. Dabei sind verschiedene Ausführungsformen beliebig miteinander kombinierbar, sofern sich aus dem Kontext nicht eindeutig das Gegenteil ergibt.

Die Nitrierung von Benzol zu Nitrobenzol mit Salpetersäure oder einem Gemisch aus Salpetersäure und Schwefelsäure (Mischsäure) in **Schritt a)** erfolgt dabei nach einem beliebigen der dem Fachmann bekannten Verfahren aus dem Stand der Technik, wie z. B. in EP 0 436 443 B1, EP 0 771 783 B1, US 6 562 247 B2 oder in EP 0 156 199 B1 beschrieben. Da in allen Verfahren des Standes der Technik ein Roh-Nitrobenzol erhalten wird, das überschüssige Säure, nicht umgesetztes Benzol, Wasser und organische Nebenkomponenten enthält, ist die erfindungsgemäße Reinigung des in Schritt a) erhaltenen Roh-Nitrobenzols grundsätzlich auf alle Verfahren anwendbar. Z. B. kann die Nitrierung unter Abfuhr der Reaktionswärme (d. h. isotherm oder angenähert isotherm) oder auch ohne Abfuhr der Reaktionswärme in bevorzugt isolierten Reaktoren (d. h. adiabat) erfolgen. Bevorzugt ist jedoch die Umsetzung von Benzol mit einem Gemisch aus Salpetersäure und Schwefelsäure unter adiabater Verfahrensführung, wie sie in DE 10 2008 048 713 A1, insbesondere Absatz [0024], beschrieben ist.

Das in Schritt a) hergestellte rohe Nitrobenzol wird zunächst in einem Trennbehälter von überschüssiger Säure (bei Verwendung von Mischsäure im Wesentlichen Schwefelsäure) getrennt. Anschließend wird die so erhaltene organische Phase, die üblicherweise noch Spuren an Säure enthält, in einer bis zwei, bevorzugt einer Wäsche(n) in **Schritt b)** mit einer wässrigen Waschflüssigkeit gewaschen und dann von der sauren wässrigen Phase durch Phasentrennung getrennt (bei mehreren Wäschen nach jeder einzelnen Wäsche). In Schritt b) werden die Säurereste, die das rohe Nitrobenzol enthält, ausgewaschen; daher wird dieser Verfahrensschritt auch als *saure Wäsche* bezeichnet. Bevorzugt wird dabei so verfahren, dass sich in der nach der Phasentrennung erhaltenen wässrigen Phase ein pH-Wert < 5 (gemessen bei 20 °C) eingestellt. Als wässrige Waschflüssigkeit kann in Schritt b) jede Art von Wasser, z. B. vollentsalztes Wasser oder Dampfkondensat, eingesetzt werden. Das Wasser kann auch gelöste Salze enthalten. Bevorzugt werden zur Durchführung von Schritt b) im Betrieb anfallende wässrige Ströme rezyklisiert.

Die so erhaltene organische Phase wird anschließend in **Schritt c)** in einer bis zwei, bevorzugt einer alkalischen Wäsche(n) mit einer wässrigen Lösung einer Base ausgewählt aus der Gruppe bestehend aus Natriumhydroxid, Natriumcarbonat und Natriumhydrogencarbonat, gewaschen und anschließend von dem alkalischen Waschwasser durch Phasentrennung getrennt (bei mehreren Wäschen nach jeder einzelnen Wäsche). Bevorzugt wird als wässrige Baselösung Natronlauge eingesetzt. Die alkalische Wäsche wird im Folgenden anhand von Natronlauge beschrieben; für den Fachmann ist es ein Leichtes, bei Einsatz anderer Basen erforderlichenfalls entsprechende Abänderungen vorzunehmen.

Bevorzugt hat die eingesetzte Natronlauge einen pH-Wert von 9,0 bis 14 (gemessen bei 20 °C). Das Massenverhältnis von Natronlauge zu organischer Phase (im Wesentlichen Nitrobenzol) ist abhängig vom in Schritt a) eingesetzten Benzolüberschuss und beträgt bevorzugt 1 : 80 bis 1 : 500. Der pH-Wert der eingesetzten Natronlauge und ihr Massenverhältnis zur organischen Phase werden so eingestellt, dass acide Verunreinigungen (z. B. als Nebenprodukte gebildete Nitrophenole und in Schritt b) nicht vollständig entfernte Säurereste) in Schritt c) weitgehend bis vollständig, bevorzugt vollständig, neutralisiert werden.

Die anschließende Aufarbeitung des alkalischen Abwassers kann nach den Verfahren des Standes der Technik, z. B. gemäß EP 1 593 654 A1 und EP 1 132 347 A2 erfolgen. Die so erhaltene organische, Nitrobenzol enthaltende Phase weist bevorzugt eine Temperatur von 20 °C bis 60 °C, besonders bevorzugt von 30 °C bis 50 °C auf. Bevorzugt enthält sie neben Nitrobenzol 4,0 bis 10 Massen-% Benzol, und weniger als 100 ppm, besonders bevorzugt weniger als 60 ppm, an Nitrophenolen, jeweils bezogen auf die Gesamtmasse der in Schritt c) erhaltenen organischen Phase.

In **Schritt d)** wird die organische Phase aus Schritt c) in einer bis zwei, bevorzugt einer Wäsche(n) mit einer wässrigen Lösung eines Kaliumsalzes gewaschen. Selbstverständlich können auch mehrere Kaliumsalze eingesetzt werden. Das Massenverhältnis von Kaliumsalz-haltigem Waschwasser zu organischer Phase beträgt bevorzugt 1 : 20 bis 1 : 1, wobei eine solche Konzentration an Kaliumionen im Kaliumsalz-haltigem Waschwasser eingestellt wird, dass die Natriumionen möglichst vollständig verdrängt werden (siehe unten für Details). Die Wäsche(n) in Schritt d) können in allen dem Fachmann bekannten Apparaten durchgeführt werden, z. B. kann dieser Waschschritt durch Dispergierung der organischen Phase mit der wässrigen Lösung eines Kaliumsalzes mittels eines Rührwerksbehälters oder eines statischen Mischers oder einer Pumpe durchgeführt werden. Die anschließende Trennung der Phasen (bei mehreren Wäschen nach jeder einzelnen Wäsche) erfolgt in Trennbehältern (Settler mit oder ohne Koaleszer) und unter Umständen unter Zuhilfenahme von Desemulgatoren (Trennhilfen).

Bevorzugt ist es, in Schritt d) ein molares Verhältnis von Kalium- zu Natriumionen von 1 : 1 bis 20 : 1, besonders bevorzugt von 2 : 1 bis 15 : 1, ganz besonders bevorzugt von 3 : 1 bis 10 : 1, einzuhalten. Selbst bei Verwendung des größten Überschusses von 20 : 1 ist die einzusetzende Menge an Kaliumsalz erheblich geringer als bei einer Verwendung von Kalilauge an Stelle von Natronlauge in der alkalischen Wäsche. In dieser Ausführungsform ist es erforderlich, Informationen über den Stoffmengenanteil an Natrium in der organischen Phase aus Schritt c) zu haben. Erfindungsgemäß maßgeblich für die Bestimmung des molaren Verhältnisses von Kaliumzu Natriumionen ist die aus dem durch Atomabsorptionspektrometrie (Inductive Coupled Plasma, ICP) bestimmten Massenanteil an Natrium berechnete Stoffmenge an Natrium. Hierzu wird eine Probe der zu untersuchenden organischen, Nitrobenzol enthaltenden Phase in Tetrahydrofuran gelöst (15,0 g der Probe werden zu 50,0 ml gelöst) und im Graphitrohrofen atomisiert. Als Vergleich werden Proben bekannten Natriumgehalts analysiert. In der betrieblichen Praxis ist es in der Regel nicht erforderlich, laufend Messungen vorzunehmen. Im Allgemeinen bilden sich nach kurzer Zeit verlässliche Erfahrungswerte für die einzusetzende Menge an Kaliumsalz(en) heraus, die nur noch von Zeit zu Zeit kontrolliert werden müssen.

Die erfindungsgemäß in Schritt d) einzusetzenden Kaliumsalze sind bevorzugt ausgewählt aus der Gruppe bestehend aus Kaliumhydroxid, Kaliumsulfat, Kaliumnitrat und Kaliumcarbonat. Besonders bevorzugt sind Kaliumhydroxid und Kaliumsulfat. Bei Verwendung von Kaliumhydroxid in Schritt d) handelt es sich theoretisch um eine der eigentlichen alkalischen Wäsche mit Natronlauge nachgeschaltete weitere "alkalische Wäsche", bei der jedoch die Basenmenge erheblich geringer ist als in Schritt c). Kaliumhydroxid dient in dieser Ausführungsform lediglich als Quelle für Kaliumionen; die Effekte der Basizität der Hydroxidanionen sind nicht ausschlaggebend.

In Schritt e) wird die in Schritt d) erhaltene organische Phase in mindestens einer, bevorzugt zwei bis vier, besonders bevorzugt zwei bis drei, ganz besonders bevorzugt zwei, neutralen Wäsche(n) mit Wasser gewaschen und anschließend von der wässrigen Phase durch Phasentrennung getrennt (bei mehreren Wäschen nach jeder einzelnen Wäsche). Dies kann grundsätzlich nach allen im Stand der Technik üblichen Verfahren geschehen. Als Waschwasser wird dabei bevorzugt vollentsalztes Wasser (VE-Wasser), besonders bevorzugt eine Mischung aus VE-Wasser und Dampfkondensat (d. i. ein Kondensat von Wasserdampf, welcher durch Wärmeaustausch von Wasser mit beliebigen exothermen Verfahrensschritten erhalten wurde) und ganz besonders bevorzugt Dampfkondensat eingesetzt.

Bevorzugt ist jedoch eine Verfahrensweise, bei der in der letzten neutralen Wäsche eine Elektrophorese eingesetzt wird. Hierzu wird in der letzten Wäsche die organische, im Wesentlichen Nitrobenzol enthaltende Phase so mit Wasser vermischt, dass sie zumindest teilweise dispergiert vorliegt. Bevorzugt enthält die Dispersion 5,0 Massen-% bis 20 Massen-% Wasser, bezogen auf die Masse der Dispersion. Dies erfolgt bevorzugt mit einem Rührer oder einem Mischer oder bevorzugt in einer Pumpe, besonders bevorzugt in einer Kreiselpumpe, deren Laufrad sich mit wenigstens 1450 und bevorzugt mit wenigstens 2900 Umdrehungen pro Minute dreht. Der Energieeintrag durch das Mischorgan liegt bevorzugt bei 20 kW / m³ bis 30 kW / m³. Als Waschwasser wird auch in dieser Ausführungsform bevorzugt vollentsalztes Wasser (VE-Wasser), besonders bevorzugt eine Mischung aus VE-Wasser und Dampfkondensat und ganz besonders bevorzugt Dampfkondensat eingesetzt. Im Anschluss an die Vermischung und Dispergierung wird die Dispersion bevorzugt einer Elektrophorese-Einheit zugeführt, worin die Dispersion ein Gleichspannungsfeld durchläuft. Dabei wird die Dispersion gebrochen, das heißt, die Phasengrenzfläche sinkt und die Phasen separieren sich wieder. In der Elektrophorese-Einheit passiert die Dispersion ein Gleichspannungsfeld von bevorzugt 100 Volt bis 500 Volt, besonders bevorzugt 200 Volt bis 400 Volt und ganz besonders bevorzugt von 220 Volt bis 300 Volt. Die Stromstärke beträgt bevorzugt 0,050 Ampere bis 3,0 Ampere und besonders bevorzugt 0,10 Ampere bis 1,0 Ampere. Durch die kontinuierliche Fahrweise des Waschprozesses wird dafür gesorgt, dass der Elektrodenraum der Elektrophorese-Einheit stets geflutet ist, so dass auch bei eventuellen elektrischen Überschlägen zwischen den Elektroden keine Gefahr einer Zündung von gegebenenfalls zündfähigen Gasgemischen im Elektrodenraum besteht. Anschließend werden die organische und die wässrige Phase in einem Trennbehälter getrennt.

In Schritt f) wird das rohe Nitrobenzol anschließend von Wasser, nicht umgesetztem Benzol und ggf. organischen Verunreinigungen durch Aufarbeitung befreit. Die Aufarbeitung erfolgt bevorzugt durch Destillation, wobei über Kopf Wasser und Benzol und ggf. organische Verunreinigungen ausgetrieben werden. Bevorzugt wird als Destillationsapparat eine Rektifizierkolonne eingesetzt. Es verbleibt das getrocknete und von Benzol befreite Nitrobenzol.

Erfindungsgemäß hergestelltes Nitrobenzol enthält bevorzugt maximal 0,40 Massen-ppm, besonders bevorzugt maximal 0,20 Massen-ppm, ganz besonders bevorzugt maximal 0,10 Massen-ppm an anorganischen Salzen als Verunreinigungen (Salze, bestimmt als Kationen, per Atomabsorptionspektrometrie (Inductive Coupled Plasma, ICP); siehe oben). Diese anorganischen Verunreinigungen bestehen dabei zu mindestens 40 Massen-%, bevorzugt zu mindestens 60 Massen-%, besonders bevorzugt zu mindestens 80 Massen-%, bezogen auf die Gesamtmasse aller anorganischen Verunreinigungen, aus Kaliumsalzen.

Ein weiterer Gegenstand der vorliegenden Erfindung ist ein Verfahren zur Herstellung von Anilin, bei dem Nitrobenzol erfindungsgemäß hergestellt und durch katalytische Hydrierung zu Anilin umgesetzt wird. Dies kann grundsätzlich nach allen dem Fachmann bekannten Verfahren des Standes der Technik erfolgen. Bevorzugt erfolgt die Anilinherstellung jedoch in der Gasphase unter Rezyklierung nicht umgesetzten Wasserstoffs (Krcisgasfahrweise), besonders bevorzugt unter Verdüsung des Nitrobenzols in den Kreisgasstrom hinein. Ganz besonders bevorzugt wird die Hydrierung gemäß dem Verfahren aus DE 10 2006 035 203 A1 durchgeführt. Insbesondere bevorzugt werden die dort in Absatz [0018] genannten Bereiche von Temperatur, Druck, Wassergehalt im Eduktgasstrom und Wasserstoffüberschuss eingehalten.

Bevorzugt wird in der Hydrierung ein Mehrkomponenten-Katalysator eingesetzt, der mindestens Palladium, Vanadium und Blei als Aktivkomponenten auf einem Träger auf α-Aluminiumoxid enthält. Diese Katalysatoren müssen in regelmäßigen Abständen mit Sauerstoff-haltigen Gasen regeneriert werden (vgl. DE 10 2006 035 203 A1, Absatz [0046]), sodass sich Produktions- und Regenerationszyklen abwechseln. Nach einer sehr langen Folge von Produktions- und Regenerationszyklen kann es jedoch vorkommen, dass die Aktivität eines Katalysators weder durch Regeneration noch durch andere Maßnahmen (bspw. Waschen) wiederherstellbar ist. Dies ist im Allgemeinen daran erkennbar, dass selbst mit frisch regeneriertem Katalysator der Umsatz an Nitrobenzol in der Hydrierung 99,7 % nicht mehr übersteigt. Es ist daher bevorzugt, denselben Katalysator nur so lange in der Hydrierung zu verwenden, solange in einem Produktionszyklus ein Umsatz an Nitrobenzol größer oder gleich 99,7 % zumindest zeitweise, bevorzugt für mindestens 80 % der Dauer des jeweiligen Produktionszyklus, erreicht wird. Der Umsatz wird dabei durch Gaschromatographie bestimmt. *"Derselbe Katalysator*" meint dabei *den konkret eingesetzten* Katalysator. Dieser wird durch Regeneration oder Waschen nicht substanziell verändert, sodass erfindungsgemäß immer noch von "demselben Katalysator" gesprochen wird. Nicht mehr "derselbe Katalysator" liegt dagegen vor, wenn die Katalysatorfüllung eines Hydrierreaktors durch eine andere Katalysatorfüllung (die durchaus zum gleichen *Typ* von Katalysator gehören kann) ersetzt wird. Wenn dies erforderlich wird, so wird der nicht mehr einsetzbare Katalysator bevorzugt so aufgearbeitet, dass die Aktivkomponenten zumindest teilweise wiedergewonnen und in der Herstellung neuen Katalysators eingesetzt werden können.

### Beispiele

### Messmethoden

Gehalt an organischen Komponenten: Gaschromatographie (GC), angegeben sind Flächen-%.
Wassergehalt: Methode nach Karl Fischer.
Gehalt an Kationen: Atomabsorptionspektrometrie (Inductive Coupled Plasma, ICP), angegeben sind Massenanteile in ppm oder ppb.

### Allgemeine Bedingungen für die Herstellung von Nitrobenzol

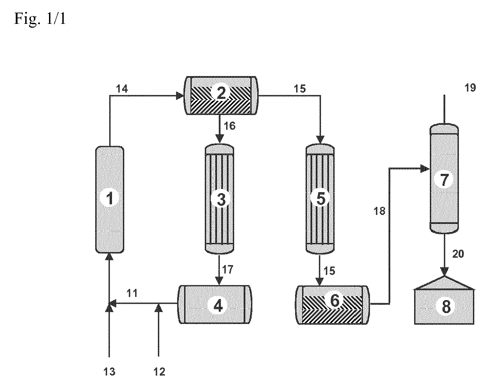

### A. Verfahren des Standes der Technik, (siehe Figur 1)

Einem Reaktor (1) werden ein Schwefelsäure- (11), ein Salpetersäure- (12) und ein Benzolstrom (13) zugeführt. Nach vollständiger Umsetzung der Salpetersäure mit dem Benzol zu Nitrobenzol unter adiabater Reaktionsführung wird das nunmehr ca. 130 °C heiße Reaktionsprodukt (14) einem Phasentrennapparat (2) zugeführt, in dem das Reaktionsprodukt (14) in eine organische Phase ((15), = Roh-Nitrobenzol, enthaltend neben Nitrobenzol noch Benzol) und eine wässrige Phase ((16), = Absäure, enthaltend neben Schwefelsäure noch geringe Anteile an Nitrobenzol und Benzol) zerfällt. Die wässrige, hauptsächlich Schwefelsäure umfassende Phase (16) wird im Verdampfer (3) durch plötzliche Druckerniedrigung einer Blitzverdampfung von Wasser unterzogen und auf diese Weise aufkonzentriert. Die aufkonzentrierte Schwefelssäure (17) wird im Schwefelsäuretank (4) zur erneuten Verwendung gelagert. Nach dem Abtrennen im Phasentrennapparat wird das Rohnitrobenzol (15) in der Rohnitrobenzolkühlung (5) abgekühlt und der Wäsche (6) zugeführt, die sich aus drei Arten von Wäschen zusammensetzt, nämlich (i) einer "sauren Wäsche" (siehe oben), (ii) einer alkalischen Wäsche mit Natronlauge (nach der in den Absätzen [0008] bis [0012] von EP 1 816 117 B1 beschriebenen Vorgehensweise) und (iii) drei neutralen Wäschen mit Dampfkondensat, wobei in der letzten neutralen Wäsche optional eine Elektrophorese (vgl. EP 1 816 117 B1) eingesetzt wird. Der so erhaltene von Nitrophenolen und Salzen weitgehend befreite Strom des gereinigten Nitrobenzols (18) wird wieder aufgeheizt und in einer Destillationskolonne (7) von Wasser und Benzol, welche über Kopf abgetrennt werden (19), befreit, wodurch getrocknetes Rein-Nitrobenzol (20) erhalten und im Tank (8) gelagert wird.

Das so erhaltene Rein-Nitrobenzol (20) wurde analysiert:

**Tabelle 1: Analysenresultate des nach dem Stand der Technik hergestellten Nitrobenzols**

| **Verfahren *A*** | **MNB^{[a]} [%]** | **Benzol [%]** | **1,3-DNB^{[b]} [%]** | **Wasser [%]** | **Na-Ionen [ppm]** | **Nitrophenole [ppm]** |
|---|---|---|---|---|---|---|
| *A.1* ohne Elektrophorese | 99,968 | 0,0018 | 0,0223 | 0,0068 | 0,317 | <5 |
| *A.2* mit Elektrophorese | 99,969 | 0,0016 | 0,0227 | 0,0060 | 0,089 | < 5 |
| *Messmethode:* | GC | GC | GC | Karl Fischer | AAS^{[c]} | HPLC |

| | | | | | | |
|---|---|---|---|---|---|---|
| [a]: Mononitrobenzol, [b] 1,3-Dinitrobenzol, [c] Atomabsorptionsspektromektrie mit elektrothermischer Aufheizung; wenn verschiedene Metalle gemessen werden sollen, wird sequentiell gemessen. | | | | | | |

### B. Erfindungsgemäßes Verfahren

In den erfindungsgemäßen Beispielen 4 und 5 wurde bis zur alkalischen Wäsche so verfahren wie unter A beschrieben. Das rohe Nitrobenzol aus der alkalischen Wäsche wurde mit Kaliumhydroxidhaltigem Wasser mittels eines statischen Mixers dispergiert (Schritt d) des erfindungsgemäßen Verfahrens). Die anschließende Phasentrennung erfolgte in einem Trennbehälter. Daran schlossen sich zwei neutrale Wäschen an.

Die Massenanteile an Natrium und Kalium wurde mittels Atomabsorptionspektrometrie (Inductive Coupled Plasma, ICP) bestimmt (siehe oben).

### Konkrete Bedingungen der Nitrobenzolherstellung in den Beispielen

In **Beispiel 1** wurde ein "salzfreies" (Restgehalt an Natrium < 15 Massen-ppb (Nachweisgrenze)) Nitrobenzol eingesetzt. Dieses wurde erhalten, indem nach der allgemeinen Vorschrift A hergestelltes Nitrobenzol über Kopf destilliert wurde. Das Vergleichsbeispiel 1 dient als Referenz sowohl für die Beispiele 2 und 3 (Nitrobenzol nach dem Verfahren des Standes der Technik, Vorschrift A, hergestellt) als auch für die Beispiele 4 und 5 (Nitrobenzol nach dem erfindungsgemäßen Verfahren, Vorschrift B, hergestellt). Ein so hergestelltes, salzfreies Nitrobenzol kennzeichnet den Idealzustand, der in der betrieblichen Praxis aufgrund der hohen Kosten einer aufwändigen Destillation des Nitrobenzols nicht erreicht wird.
In **Beispiel 2** wurde ein Nitrobenzol eingesetzt, das 89 Massen-ppb (0,089 Massen-ppm) Natrium enthielt. Dieses wurde erhalten, indem Nitrobenzol nach dem allgemeinen Nitrierverfahren A unter Verwendung einer Elektrophorese hergestellt wurde. Beispiel 2 dient als Vergleichsversuch, der die Verfahren des Standes der Technik repräsentiert, in denen die alkalische Wäsche mit Natronlauge durchgeführt wird und der erfindungsgemäße Schritt d) fehlt.
In **Beispiel 3** wurde ein Nitrobenzol eingesetzt, das 317 Massen-ppb (0,317 Massen-ppm) Natrium enthielt. Dieses wurde erhalten, indem Nitrobenzol nach dem allgemeinen Nitrierverfahren A ohne Verwendung einer Elektrophorese hergestellt wurde. Beispiel 3 dient als Vergleichsversuch, der die Verfahren des Standes der Technik repräsentiert, in denen die alkalische Wäsche mit Natronlauge durchgeführt wird und der erfindungsgemäße Schritt d) fehlt.
In **Beispiel 4** wurde der Natriumgehalt der organischen Phase nach der alkalischen Wäsche mit Natronlauge zu 10 Massen-ppm bestimmt. Die organische Phase wurde nun wie unter *B* beschrieben mit einer wässrigen Lösung von KOH gewaschen (85 mg KOH pro kg organische Phase, entsprechend einem fünffachen molaren Überschuss von Kalium). In den neutralen Wäschen wurde keine Elektrophorese eingesetzt. Nach den neutralen Wäschen enthielt das Nitrobenzol 44 Massen-ppb Natrium und 311 Massen-ppb Kalium. Der Natriumgehalt konnte also *ohne* Elektrophorese gegenüber dem Verfahren nach dem Stand der Technik *mit* Elektrophorese halbiert werden (vgl. Tabelle 1, VerfahrenA.2). Beispiel 4 ist erfindungsgemäß.
In **Beispiel 5** wurde der Natriumgehalt der organischen Phase nach der alkalischen Wäsche mit Natronlauge zu 10 Massen-ppm bestimmt. Die organische Phase wurde nun wie unter *B* beschrieben mit einer wässrigen Lösung von KOH gewaschen (170 mg KOH pro kg organische Phase, entsprechend einem zehnfachen molaren Überschuss von Kalium). In der letzten neutralen Wäsche wurde eine Elektrophorese eingesetzt. Nach den neutralen Wäschen enthielt das Nitrobenzol < 15 Massen-ppb (unterhalb der Nachweisgrenze) Natrium und 115 Massen-ppb Kalium. Beispiel 5 ist erfindungsgemäß.

### Allgemeine Bedingungen für die Hydrierung von Nitrobenzol zu Anilin (in allen Beispielen eingehalten)

Als Versuchsanlage für die Beispielreaktionen dient ein 500 mm langes Reaktionsrohr aus Edelstahl. Durch diesen Reaktor wird ein Kreisgasstrom geleitet, der mittels eines Wärmeaustauschers auf 250 °C erwärmt wird. Mittels Dosierpumpen wird Nitrobenzol zu einer Düse gefördert und im Kreisgasstrom fein zerstäubt, wo es dann verdampft. Wasserstoff wird in einem Wärmeaustauscher vorerwärmt und dem Kreisgas vor dieser Düse zudosiert. Die Wasserstoff-Versorgung wird durch einen Massedurchflussregler geregelt. Die Belastung des im Reaktionsrohr befindlichen Katalysators wird in allen Beispielversuchen auf einen Wert von 1,0 g_{Nitrobenzol}/(ml_{Katalysator}· h) eingestellt ("ml_{Katalysator}" bezieht sich auf das Schüttvolumen des Katalysators), und das Wasserstoff : Nitrobenzol-Verhältnis im Reaktor wurde auf ca. 80 : 1 festgelegt.

Auf einem Sieb innerhalb des Reaktionsrohrs wird eine 400 mm hohe Schüttung des Katalysators platziert. Nach Austritt des Reaktors wird das Reaktionsprodukt mit Wasser gekühlt. Die nicht flüchtigen Bestandteile werden so auskondensiert und in einem nachgeschalteten Abscheider von den gasförmigen Komponenten getrennt. Die flüssigen Bestandteile werden aus dem Abscheider in den Produktsammelbehälter geführt und dort aufgefangen. Vor dem Sammelbehälter befindet sich eine Probennahmestelle, an der in regelmäßigen Zeitabständen Proben des Produkts gezogen werden können. Diese werden gaschromatographisch analysiert. Die Standzeit des Katalysators entspricht der Zeit vom Beginn der Reaktion bis kein vollständiger Umsatz des Nitrobenzols mehr erreicht wird und an der Probennahmestelle > 0,1 % Nitrobenzol im Produkt mittels Gaschromatographie detektiert werden.

Die Beispiele wurden mit dem Katalysatorsystem 9 g/ l_{Träger} Pd, 9 g/ l_{Träger} V, 3 g/ l_{Träger} Pb auf α-Aluminiumoxiddurchgeführt (siehe EP 0 011 090 A1).

Frisch hergestellter Katalysator wurde jeweils im Reaktionsrohr platziert und zunächst mit Stickstoff, dann mit Wasserstoff gespült. Im Anschluss wurde der Katalysator über einen Zeitraum von 48 h bei 240 °C mit 1000 l/h Wasserstoff beaufschlagt. Dann wurde verdampftes Nitrobenzol auf den Katalysator geleitet. Die Nitrobenzolbelastung wurde langsam auf den gewünschten Wert von 1,0 g_{Nitrobenzol}/(ml_{Katalysator} ·h) erhöht, sodass die Temperatur im Reaktor nicht über 450 °C anstieg, und die Wasserstoffzugabe wurde so eingestellt, dass das molare Verhältnis von Wasserstoff: Nitrobenzol 80 : 1 betrug. Sobald kein vollständiger Umsatz von Nitrobenzol mehr erfolgte (mehr als 0,1 % Nitrobenzol im Reaktionsprodukt), wurde die Edukteinspeisung beendet und der Reaktor mit Stickstoff inertisiert. Bei 270 °C wurden dann im Luftstrom Verkokungen abgebrannt, bis im Abgas weniger als 0,2 Vol.-% CO₂ detektiert werden konnten. Dieser Zyklus aus Produktion und Regeneration des Katalysators wurde jeweils dreimal durchgeführt.

Die folgende Tabelle 2 stellt die Resultate der Beispiele 1 bis 5 gegenüber:

**Tabelle 2**

| **Beispiel Nr.:** | **1 (Vgl., Ref.)** | **2 (Vgl.)** | **3 (Vgl.)** | **4 (erf.)** | **5 (erf.)** |
|---|---|---|---|---|---|
| | *Standzeit in Stunden* | | | | |
| *im 1. Produktionszyklus:* | 983 | 953 | 946 | 978 | 981 |
| *im 2. Produktionszyklus:* | 964 | 902 | 804 | 957 | 982 |
| *im 3. Produktionszyklus:* | 968 | 850 | 675 | 943 | 972 |

| | | | | | |
|---|---|---|---|---|---|
| Ref. = Referenzversuch; Vgl. = Vergleichsversuch; erf. = erfindungsgemäßer Versuch | | | | | |

Wie der Tabelle zu entnehmen ist, sind die Standzeiten in den erfindungsgemäßen Beispielen ähnlich gut oder sogar besser als im Referenzversuch. In den Vergleichsbeispielen ohne den erfindungsgemäßen Schritt d) sind die Standzeiten dagegen im Vergleich zum Referenzversuch signifikant verkürzt. Je größer der Natriumgehalt im Nitrobenzol, desto schlechter ist die Standzeit.

## Patentansprüche

1. Verfahren zur Herstellung von Nitrobenzol durch
a) Nitrierung von Benzol mit Salpetersäure oder Gemischen aus Salpetersäure und Schwefelsäure und anschließende Phasentrennung in eine wässrige und eine organische, Nitrobenzol enthaltende Phase,
b) Waschen der in Schritt a) erhaltenen organischen, Nitrobenzol enthaltenden Phase in mindestens einer Wäsche(n) und anschließende Phasentrennung in eine wässrige und eine organische, Nitrobenzol enthaltende Phase,
c) Waschen der in Schritt b) erhaltenen organischen, Nitrobenzol enthaltenden Phase in mindestens einer alkalischen Wäsche(n) mit einer wässrigen Lösung einer Base ausgewählt aus der Gruppe bestehend aus
Natriumhydroxid, Natriumcarbonat und Natriumhydrogencarbonat,
und anschließende Phasentrennung in eine wässrige und eine organische, Nitrobenzol enthaltende Phase,
d) Waschen der in Schritt c) erhaltenen organischen, Nitrobenzol enthaltenden Phase in mindestens einer Wäsche(n) mit einer wässrigen Lösung eines Kaliumsalzes und anschließende Phasentrennung in eine wässrige und eine organische, Nitrobenzol enthaltende Phase,
e) Waschen der in Schritt d) erhaltenen organischen, Nitrobenzol enthaltenden Phase in mindestens einer neutralen Wäsche(n) mit Wasser und anschließende Phasentrennung in eine wässrige und eine organische, Nitrobenzol enthaltende Phase,
f) Aufarbeitung der in Schritt e) erhaltenen, organischen, Nitrobenzol enthaltenden Phase, wobei gereinigtes Nitrobenzol erhalten wird.

2. Verfahren nach Anspruch 1, bei dem in Schritt c) eine wässrige Lösung von Natriumhydroxid eingesetzt wird.

3. Verfahren nach Anspruch 1 oder 2, bei dem in Schritt d) ein molares Verhältnis von Kaliumzu Natriumionen von 1 : 1 bis 20 : 1 eingehalten wird.

4. Verfahren nach einem der Ansprüche 1 bis 3, bei dem in der letzten neutralen Wäsche in Schritt e) eine Elektrophorese eingesetzt wird.

5. Verfahren nach einem der Ansprüche 1 bis 4, bei dem das in Schritt d) eingesetzte Kaliumsalz ausgewählt ist aus der Gruppe bestehend aus Kaliumhydroxid, Kaliumsulfat, Kaliumcarbonat und Kaliumnitrat.

6. Verfahren nach Anspruch 5, bei dem das in Schritt d) eingesetzte Kaliumsalz Kaliumhydroxid ist.

7. Verfahren nach Anspruch 5, bei dem das in Schritt d) eingesetzte Kaliumsalz Kaliumsulfat ist.

8. Verfahren zur Herstellung von Anilin, bei dem man Nitrobenzol durch Nitrierung von Benzol gemäß einem der Ansprüche 1 bis 7 herstellt und dieses Nitrobenzol in Gegenwart eines Katalysators zu Anilin hydriert.

9. Verfahren nach Anspruch 8, bei dem als Katalysator ein Mehrkomponenten-Katalysator eingesetzt wird, der mindestens Palladium, Vanadium und Blei als Aktivkomponenten auf einem Träger auf α-Aluminiumoxid enthält.

10. Verfahren nach Anspruch 9, bei dem derselbe Katalysator nur so lange eingesetzt wird, solange in einem Produktionszyklus ein Umsatz an Nitrobenzol größer oder gleich 99,7 % zumindest zeitweise erreicht wird.

11. Verfahren nach Anspruch 10, bei dem der nicht mehr eingesetzte Katalysator so aufgearbeitet wird, dass die Aktivkomponenten zumindest teilweise wiedergewonnen und in der Herstellung neuen Katalysators eingesetzt werden.

## Claims

1. Method for producing nitrobenzene by
a) nitration of benzene with nitric acid or mixtures of nitric acid and sulfuric acid and subsequent phase separation into an aqueous phase and an organic phase containing nitrobenzene,
b) washing of the organic nitrobenzene-containing phase obtained in step a) in at least one wash and subsequent phase separation into an aqueous phase and an organic phase containing nitrobenzene,
c) washing of the organic nitrobenzene-containing phase obtained in step b) in at least one alkaline wash with an aqueous solution of a base selected from the group consisting of
sodium hydroxide, sodium carbonate and sodium hydrogen carbonate,
and subsequent phase separation into an aqueous phase and an organic phase containing nitrobenzene,
d) washing of the organic nitrobenzene-containing phase obtained in step c) in at least one wash with an aqueous solution of a potassium salt and subsequent phase separation into an aqueous phase and an organic phase containing nitrobenzene,
e) washing of the organic nitrobenzene-containing phase obtained in step d) in at least one neutral wash with water and subsequent phase separation into an aqueous phase and an organic phase containing nitrobenzene,
f) processing of the organic nitrobenzene-containing phase obtained in step e), wherein purified nitrobenzene is obtained.

2. Method according to claim 1, wherein in step c) an aqueous solution of sodium hydroxide is used.

3. Method according to claim 1 or 2, wherein in step d) a molar ratio of potassium to sodium ions of 1:1 to 20:1 is maintained.

4. Method according to one of claims 1 to 3, wherein in step e) electrophoresis is used in the last neutral wash.

5. Method according to one of claims 1 to 4, wherein the potassium salt used in step d) is selected from the group consisting of potassium hydroxide, potassium sulfate, potassium carbonate and potassium nitrate.

6. Method according to claim 5, wherein the potassium salt used in step d) is potassium hydroxide.

7. Method according to claim 5, wherein the potassium salt used in step d) is potassium sulfate.

8. Method for producing aniline, wherein nitrobenzene is produced by nitration of benzene according to one of claims 1 to 7 and said nitrobenzene is hydrogenated to aniline in the presence of a catalyst.

9. Method according to claim 8, wherein a multicomponent catalyst containing at least palladium, vanadium and lead as active components on a support on α-aluminium oxide is used as the catalyst.

10. Method according to claim 9, wherein the same catalyst is used only for as long as a conversion to nitrobenzene of greater than or equal to 99.7% is achieved in a production cycle for at least some of the time.

11. Method according to claim 10, wherein the catalyst that is no longer used is processed in a manner such that the active components are at least partly recovered and used in the production of new catalyst.

## Revendications

1. Procédé de fabrication de nitrobenzène par
a) la nitration de benzène avec de l'acide nitrique ou des mélanges d'acide nitrique et d'acide sulfurique, puis la séparation de phases en une phase aqueuse et une phase organique contenant du nitrobenzène,
b) le lavage de la phase organique contenant du benzène obtenue à l'étape a) dans au moins un lavage, puis la séparation de phases en une phase aqueuse et une phase organique contenant du nitrobenzène,
c) le lavage de la phase organique contenant du nitrobenzène obtenue à l'étape b) dans au moins un lavage alcalin avec une solution aqueuse d'une base choisie dans le groupe constitué par
l'hydroxyde de sodium, le carbonate de sodium et l'hydrogénocarbonate de sodium,
puis la séparation de phases en une phase aqueuse et une phase organique contenant du nitrobenzène,
d) le lavage de la phase organique contenant du nitrobenzène obtenue à l'étape c) dans au moins un lavage avec une solution aqueuse d'un sel de potassium, puis séparation de phases en une phase aqueuse et une phase organique contenant du nitrobenzène,
e) le lavage de la phase organique contenant du nitrobenzène obtenue à l'étape d) dans au moins un lavage neutre, puis la séparation de phases en une phase aqueuse et une phase organique contenant du nitrobenzène,
f) le traitement de la phase organique contenant du nitrobenzène obtenue à l'étape e), du nitrobenzène purifié étant obtenu.

2. Procédé selon la revendication 1, dans lequel, à l'étape c), une solution aqueuse d'hydroxyde de sodium est utilisée.

3. Procédé selon la revendication 1 ou 2, dans lequel, à l'étape d), un rapport molaire entre les ions potassium et sodium de 1:1 à 20:1 est maintenu.

4. Procédé selon l'une quelconque des revendications 1 à 3, dans lequel une électrophorèse est utilisée dans le dernier lavage neutre à l'étape e).

5. Procédé selon l'une quelconque des revendications 1 à 4, dans lequel le sel de potassium utilisé à l'étape d) est choisi dans le groupe constitué par l'hydroxyde de potassium, le sulfate de potassium, le carbonate de potassium et le nitrate de potassium.

6. Procédé selon la revendication 5, dans lequel le sel de potassium utilisé à l'étape d) est l'hydroxyde de potassium.

7. Procédé selon la revendication 5, dans lequel le sel de potassium utilisé à l'étape d) est le sulfate de potassium.

8. Procédé de fabrication d'aniline, dans lequel du nitrobenzène est fabriqué par nitration de benzène selon l'une quelconque des revendications 1 à 7, et ce nitrobenzène est hydrogéné en aniline en présence d'un catalyseur.

9. Procédé selon la revendication 8, dans lequel un catalyseur à plusieurs composants est utilisé en tant que catalyseur, qui contient au moins du palladium, du vanadium et du plomb en tant que composants actifs sur un support en oxyde d'aluminium α.

10. Procédé selon la revendication 9, dans lequel le même catalyseur n'est utilisé que tant qu'une conversion de nitrobenzène supérieure ou égale à 99,7 % est atteinte au moins temporairement dans un cycle de production.

11. Procédé selon la revendication 10, dans lequel le catalyseur qui n'est plus utilisé est traité de sorte que les composants actifs soient au moins partiellement récupérés et utilisés dans la fabrication de nouveaux catalyseurs.
